# EUROPÄISCHE PATENTSCHRIFT

(11) **EP 3 440 423 B1**
(45) Veröffentlichungstag und Bekanntmachung des Hinweises auf die Patenterteilung: **18.08.2021**
(21) Anmeldenummer: 16812725.6
(22) Anmeldetag: 14.12.2016
(51) Int. Cl.: F28F 21/06, A61M 1/16, B29C 48/09, B29C 48/90, B29C 48/92

(54) **VERFAHREN ZUM HERSTELLEN EINES SCHLAUCHKÖRPERS FÜR EINEN WÄRMETAUSCHERSCHLAUCH**
METHOD FOR MANUFACTURING A TUBE BODY FOR A HEAT EXCHANGER TUBE
MÉTHODE DE FABRICATION D'UN CORPS DE TUBE POUR TUBE D'ÉCHANGEUR DE CHALEUR

(30) Priorität: 17.12.2015 DE 102015225555
(43) Veröffentlichungstag der Anmeldung: 13.02.2019
(73) Patentinhaber: Raumedic AG, 95213 Münchberg (DE)
(72) Erfinder: DOLLA, Andreas, 95030 Hof (DE)
(74) Vertreter: Rau, Schneck & Hübner Patentanwälte Rechtsanwälte PartGmbB
(86) Internationale Anmeldenummer: PCT/EP2016/080886
(87) Internationale Veröffentlichungsnummer: WO 2017/102785

(56) Entgegenhaltungen:
- WO-A1-01/32398
- DE-T2- 68 925 291
- DE-T2-602004 006 097
- GB-A- 1 185 430
- US-A- 4 520 672
- US-A- 5 498 377
- US-A- 5 545 151
- US-A- 5 578 267
- US-A- 5 630 982
- US-A1- 2005 080 507
- US-A1- 2010 119 642
- US-A1- 2012 055 576
- US-A1- 2013 327 428
- Lubrizol: "Technical Data Sheet of Estane 58091 TPU", , 10. Juni 2014 (2014-06-10), XP055344368, Gefunden im Internet: URL:https://www.lubrizol.com/-/media/Lubri zol/Engineered-Polymers/Documents/TDS/Esta ne-58091.pdf [gefunden am 2017-02-09]
- Dsm: "Prdoduct Data Sheet of Elasthane TPU", , 1. Januar 2012 (2012-01-01), XP055344369, Gefunden im Internet: URL:https://www.dsm.com/content/dam/dsm/me dical/en_US/documents/elasthane(tm)-tpu-pr oduct-sheet.pdf [gefunden am 2017-02-09]
- Anil K. Bhowmick; Howard L. Stephens: "Thermoplastic Polyurethane Elastomers" In: "Handbook of Elastomers, Second edition, Revised and Expanded", 1. Januar 2000 (2000-01-01), Marcel Dekker, Inc., XP055316144, ISBN: 978-0-8247-0383-7 Seiten 2pp, 387-415, Tabellen 9, 10
- "Tolerance, chapter 6 Plastic Performance" In: Rosato, Dominick V.: "Plastics engineered product design", 2003, ELSEVIER, Oxford, XP040425525, ISBN: 1856174166 pages 414-421,

## Beschreibung

Die vorliegende Patentanmeldung nimmt die Priorität der deutschen Patentanmeldung DE 10 2015 225 555.7 in Anspruch.

Die Erfindung betrifft ein Verfahren zum Herstellen eines Schlauchkörpers für einen Wärmetauscherschlauch, einen Wärmetauscherschlauch für einen Wärmetauscher eines Oxygenators, einen Wärmetauscherkörper für einen Wärmetauscher eines Oxygenators mit einer Mehrzahl von Schlauchabschnitten aus einem derartigen Wärmetauscherschlauch und einen Wärmetauscher für einen Oxygenator mit einem derartigen Wärmetauscherkörper.

Ein Oxygenator ist ein medizinisches Produkt, das Blut mit Sauerstoff anreichert und Kohlendioxid aus dem Blut entfernt. Der Oxygenator wird in der Herzchirurgie als Teil der Herz-Lungen-Maschine verwendet und ersetzt beispielsweise während einer Operation am offenen Herzen die Funktion der Lunge. An dem Oxygenator ist zusätzlich ein Wärmetauscher für einen Wärmeaustausch zwischen Blut und einem Wärmetauschermedium, insbesondere Wasser, vorgesehen.

Ein derartiger Oxygenator mit einer Mehrzahl von Abschnitten eines Wärmetauscherschlauchs ist bekannt aus der WO 2011/086 010 A1. Weitere Oxygenatoren sind bekannt aus der US 5,876,667 und aus der DE 689 25 291 T2.

Die DE 689 25 291 T2 offenbart einen zylindrischen Blutwärmer und Blutoxygenator. Die DE 10 2012 204 705 A1 offenbart einen Wärmetauscher für einen Oxygenator.

Am 09.02.2017 war im Internet abrufbar ein Datenblatt "Estane® 58091 TPU" des Unternehmens Lubrizol Advanced Materials, Inc. Bei diesem Material handelt es sich um ein thermoplastisches Polyurthan auf Polyester-Basis.

Die US 2005/0080507 A1 offenbart eine Vorrichtung und ein Verfahren zur Extrusion.

Die US 2013/0327428 A1 beschreibt einen Schlauch aus thermoplastischem Elastomer sowie ein Verfahren zu dessen Herstellung.

Am 09.02.2017 war im Internet abrufbar ein Datenblatt "Elasthane™ - Thermoplastic Polyether Polyurethane (TPU)" des Unternehmens DSM Biomedical Inc. Das Material wird als biomedizinisches Polymer beworben.

Die US 2012/0055576 A1 offenbart ein Produkt aufweisend ein Copolymer aus thermoplastischem Polyurethan und Polyamid 6/66.

Es ist eine Aufgabe der vorliegenden Erfindung, einen Wärmetauscherschlauch für dessen Einsatz in einem Wärmetauscher eines Oxygenators zu optimieren.

Diese Aufgabe ist erfindungsgemäß gelöst durch ein Verfahren zum Herstellen eines Schlauchkörpers für einen Wärmetauscherschlauch mit den in Anspruch 1 angegebenen Schritten. Beim Herstellungsverfahren führt der Filtrationsschritt zum Herausfiltern von Verunreinigungen, so dass Inhomogenitäten im Schlauchmaterial vermieden sind. Als Gelteilchen werden vernetzte und teilvernetzte Polyurethan-Bestandteile bezeichnet. Das Ausfiltern von in der Schmelze enthaltenen Gelteilchen führt zu einer Säuberung des in der Schmelze vorliegenden Polymers von arteigenen Polymerbestandteilen, die im Rahmen der Polymerherstellung entstehen. Derartige, herausgefilterte Bestandteile können zu Unhomogenitäten im extrudierten Schlauchmaterial führen, also zu unerwünschten Schwachstellen in Bezug auf elektrische und/oder physikalische Eigenschaften. Gelteilchen können als Keime zur unerwünschten Ausbildung kristalliner Strukturen fungieren. Gelteilchen mit einer Dichte, die sich von derjenigen des sonstigen Polyurethan-Materials des Schlauchkörpers unterscheidet, führen zur unerwünschten Ausbildung von Fließzonen im Polymer. Derartige Fließ-Inhomogenitäten führen ebenfalls zu einer unerwünschten Reduzierung der Durchschlagsfestigkeit. Aufgrund des Ausfilterns der Gelteilchen erhöht sich der Anteil amorphen Materials in der Kunststoffschmelze. Dies führt zu einer Erhöhung der elektrischen Durchschlagsfestigkeit.

Beim Herstellverfahren kann der Abkühlschritt so schnell erfolgen, dass im Schlauchmaterial amorphe Materialbereiche erzeugt werden. Dies führt zu einer vorteilhaften Erhöhung der elektrischen Durchschlagsfestigkeit. Eine Abkühlrate bei dem Abkühlschritt kann größer sein als 5 K/min, kann größer sein als 10 K/min, kann größer sein als 15 K/min, kann größer sein als 20 K/min, kann größer sein als 25 K/min, kann größer sein als 30 K/min, kann größer sein als 35 K/min, kann größer sein als 40 K/min, kann größer sein als 45 K/min und kann größer sein als 50 K/min.

Die Aufgabe wird ebenfalls gelöst durch einen Wärmetauscherschlauch mit den im Anspruch 2 angegebenen Merkmalen.

Erfindungsgemäß wurde zunächst erkannt, dass ein Schlauchkörper aus einem thermoplastischen Polyurethan Vorteile gegenüber anderen Polymermaterialien hat, die im Stand der Technik zum Einsatz in Wärmetauschern eines Oxygenators vorgeschlagen werden. TPU hat gute Wärmeleitfähigkeitseigenschaften, insbesondere einen hohen Wärmeübergangskoeffizienten. TPU ist hydrophil, so dass keine elektrostatische Aufladung des Wärmetauscherschlauchs resultiert. Der Wärmetauscherschlauch hat eine hohe Durchschlagsfestigkeit. Der Wärmetauscherschlauch aus TPU hat eine gute Blutkompatibilität. Diese Vorteile gelten insbesondere für den TPU-Schlauchkörper mit der Shore-Härte > 60D. Dieser hat auch eine geringe Klebrigkeit, was zu einem guten Handling bei der Verarbeitung des Wärmetauscherschlauches führt. Der Wärmetauscherschlauch ist steif und hat eine gute Druck- und Verformungsstabilität. Bei dem TPU des Schlauchköpers kann es sich um Polyether-basierendes TPU-Material handeln. Bei dem TPU-Material des Schlauchkörpers kann es sich um aromatisches TPU handeln. Es resultiert ein hydrolysestabiler Schlauchkörper, was insbesondere Vorteile im Zusammenhang mit einem Wasser/Blut-Kontakt des Schlauchkörpers bietet. Die geringe Wandstärkenabweichung des Wärmetauscherschlauchs führt zur Möglichkeit, einen hinsichtlich seiner Wärmeübergangseigenschaften sehr homogenen Wärmetauscher zu realisieren. Die Abweichung der Wandstärke von der Sollwandstärke kann höchstens 0,015 mm oder höchstens 0,010 mm betragen. Die genannten maximalen Wandstärke-Abweichungen können auch über wesentlich größere Schlauchlängen im Bereich von mehr als 1 m, im Bereich von mehr als 10 m, im Bereich von mehr als 100 m, im Bereich von mehr als 1.000 m oder auch im Bereich von mehr als 10.000 m erhalten bleiben, so dass auch über diese größeren Schlauchlängen keine größere Abweichung der Wandstärke von einer Soll-Wandstärke als die vorstehend genannten maximalen Abweichungen vorliegt.

Shore-Härten nach den Ansprüchen 3 und 4 führen zu einem besonders vorteilhaften Wärmetauscherschlauch. Ein Beispiel für ein Schlauchkörper-Material mit einer Shore-Härte im Bereich von 73D ist Elastollan® 1174D. Ein Beispiel für ein Schlauchkörper-Material mit einer Shore-Härte im Bereich von 75D ist Tecothane™ 1075D. Ein Beispiel für ein Schlauchkörper-Material mit einer Shore-Härte im Bereich von 76D ist Pellethane® 2363 75D. Beispiele für Schlauchkörper-Materialien mit Shore-Härten im Bereich von 80D sind Tecoplast®TP-470 mit einer Shore-Härte von 82D und Isoplast®300ETPU mit einer Shore-Härte von 83D. Ein Beispiel für ein Schlauchkörper-Material mit einer Rockwell-Härte im Bereicht 121R ist Isoplast® 2530.

Die Wandstärke kleiner als 0,07 mm ergibt einen Wärmetauscherschlauch mit sehr hoher Wärmeleitfähigkeit und damit einem effektiven Wärmeübergang zwischen einem Schlauchlumen und einer Schlauchumgebung. Eine geringe Wandstärke führt zudem dazu, dass das Risiko einer Einbettung größerer Materialeinschlüsse, insbesondere von Gelteilchen, sowie des Vorhandenseins von Inhomogenitäten in der Schlauchwand reduziert ist. Es ergeben sich insgesamt homogenere Eigenschaften des Wärmetauscherschlauchs. Die Wandstärke kann beispielsweise 0,06 mm oder 0,05 mm betragen.

Die Durchschlagsfestigkeit von mindestens 10 kV führt zu einer hohen Betriebssicherheit eines mit dem Wärmetauscherschlauch ausgerüsteten Wärmetauschers. Die Durchschlagsfestigkeit ergibt sich bei einer Messung gemäß DIN EN 60243-1. Bei der Messung der Durchschlagsfestigkeit kommt ein Schlauchkörper mit einer Wandstärke zum Einsatz, die höchstens 0,1 mm beträgt und die insbesondere im Bereich zwischen 0,06 mm und 0,085 mm, beispielsweise im Bereich zwischen 0,065 und 0,080 mm, im Bereich zwischen 0,060 und 0,075 mm, im Bereich zwischen 0,077 und 0,082 mm oder im Bereich zwischen 0,075 und 0,085 mm liegt.

Die Vorteile eines Wärmetauscherkörpers nach Anspruch 5 und eines Wärmetauschers nach Anspruch 6 entsprechen denen, die vorstehend unter Bezugnahme auf den erfindungsgemäßen Wärmetauscherschlauch bereits genannt wurden.

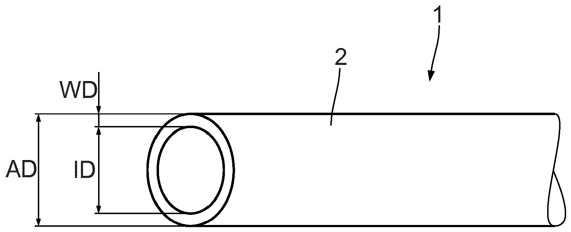

Ein Ausführungsbeispiel der Erfindung wird nachfolgend anhand der Zeichnung näher erläutert. Die einzige Fig. zeigt perspektivisch einen gebrochenen Abschnitt eines Wärmetauscherschlauches für einen Wärmetauscher eines Oxygenators.

Eine Wärmetauscherschlauch 1 ist Bestandteil eines ansonsten nicht dargestellten Wärmetauscherkörpers eines Wärmetauschers eines Oxygenators. Der Wärmetauscherschlauch 1 kann Bestandteil eines Oxygenators sein, wie er ansonsten beschrieben ist in der WO 2011/086 010 A1. Der Oxygenator hat einen rohrförmigen Grundkörper, in dem der Wärmetauscherkörper angeordnet ist.

Der Wärmetauscherschlauch hat einen Schlauchkörper 2 aus thermoplastischem Polyurethan (TPU). Der Schlauchkörper 2 hat eine Shore-Härte, die größer ist als 60D. Beispiele für ein derartiges Material sind Isoplast®300ETPU mit einer Shore-Härte von 83D und Tecoplast® TP-470 mit einer Shore-Härte von 82D.

Bei dem thermoplastischen Polyurethan des Schlauchkörpers handelt es sich um ein Polyether-basierendes TPU. Bei dem Material des Schlauchkörpers 2 handelt es sich um ein aromatisches TPU.

Der Schlauchkörper 2 hat einen Innendurchmesser ID im Bereich zwischen 0,40 mm und 0,80 mm und einen größeren Außendurchmesser AD im Bereich zwischen 0,50 mm und 1,0 mm. Eine Wandstärke WD ist beim Schlauchkörper 2 kleiner als 0,07 mm. Beispielhafte Abmessungen des Schlauchkörpers 2 sind: ID = 0,67 mm, AD = 0,79 mm, was zu einer Wandstärke WD von 0,06 mm führt, oder ID = 0,50 mm, AD = 0,6 mm, was zu einer Wandstärke WD von 0,05 mm führt.

Über eine Schlauchlänge von beispielsweise 200 mm weicht die Wandstärke WD um weniger als 0,02 mm von einer Soll-Wandstärke ab. Tatsächlich kann die Fertigung so präzise erfolgen, dass die Wandstärke WD auch über eine Schlauchlänge von mehr als 1 m, von mehr als 10 m, von mehr als 100 m, von mehr als 1.000 m und auch von mehr als 10.000 m um weniger als 0,02 mm von einer Soll-Wandstärke abweicht.

Der Wärmetauscherschlauch 1 hat eine elektrische Durchschlagsfestigkeit (electrical strength), die mindestens 10 kV beträgt und die beispielsweise im Bereich von 10 kV, 11 kV, 12 kV oder 14 kV liegt. Zur Messung der elektrischen Durchschlagsfestigkeit wird in das Lumen des Schlauchkörpers 2 eine Drahtelektrode eingeführt und eine Außenelektrode in Form einer Folie mit einer Außenwand des Schlauchkörpers 2 kontaktiert. An die beiden Elektroden wird dann eine elektrische Spannung angelegt und kontinuierlich erhöht, bis ein Durchschlag erfolgt. Diese Messung der Durchschlagsfestigkeit erfolgt entsprechend DIN EN 60243-1. Ein Wert von 14 kV für die Durchschlagsfestigkeit kann bei einer größeren Wandstärke WD beispielsweise von 0,06 mm erreicht werden. Ein Wert von 10 kV für die Durchschlagsfestigkeit kann bei einem kleineren Wert der Wandstärke WD beispielsweise von 0,05 mm erreicht werden. Für eine Wandstärke WD im Bereich von 0,065 mm wurde im Rahmen einer Messreihe eine Durchschlagsfestigkeit von 12 kV gemessen. Für eine Wandstärke WD von 0,06 mm wurde im Rahmen der Messreihe eine Durchschlagsfestigkeit von 10 kV gemessen.

Der Schlauchkörper 2 ist für sichtbares Licht transparent. Der Schlauchkörper 2 ist im Wesentlichen frei von Einschlüssen.

Bei der Herstellung des Schlauchkörpers 2 wird zunächst eine TPU-Kunststoffschmelze hergestellt.

Diese Kunststoffschmelze wird anschließend filtriert. Die Filtrierung dient dem Ausfiltern von in der Schmelze enthaltenen Gelteilchen. Die filtrierte Schmelze wird dann zu einem Schlauch extrudiert. Der extrudierte Schlauch wird abgekühlt. Dies erfolgt durch Durchführen des extrudierten Schlauches durch eine Temperierkammer, enthaltend ein Wärmeübertragungsmedium, beispielsweise durch Durchleiten des extrudierten Schlauches durch ein Wasserbecken, wobei das Wasser auf eine Temperatur im Bereich zwischen 5° und 15°, beispielsweise auf eine Temperatur von 10°, gehalten wird.

Diese Materialabkühlung erzeugt amorphe Materialbereiche im Schlauchkörper 2.

Nach dem Abkühlen wird der so hergestellte Schlauch zu für den Aufbau des Wärmetauscherkörpers erforderlichen Schlauchabschnitten konfektioniert und die Schlauchabschnitte werden dann zum Wärmetauscherkörper und zum Wärmetauscher montiert. Der Wärmetauscher ist die Hauptkomponente des Oxygenators. Diesbezüglich wird auf die WO 2011/086 010 A1 verwiesen.

## Patentansprüche

1. Verfahren zum Herstellen eines Schlauchkörpers (2) für einen Wärmetauscherschlauch (1) mit folgenden Schritten:
- Herstellen einer thermoplastischen Polyurethan Kunststoffschmelze,
- Filtrieren der Kunststoffschmelze zum Ausfiltern von in der Schmelze enthaltenen Gelteilchen,
- Extrudieren der Schmelze zu einem Schlauch,
- Abkühlen des extrudierten Schlauchs,
- Konfektionieren des Schlauchkörpers (2),
wobei der Schlauchkörper (2) eine Shore-Härte aufweist, die größer ist als 60D,
mit einer Wandstärke (WD), die über eine Schlauchlänge von 30 cm um weniger als 0,02 mm von einer Soll-Wandstärke abweicht.

2. Wärmetauscherschlauch (1) für einen Wärmetauscher eines Oxygenators, hergestellt gemäß dem Verfahren nach Anspruch 1,
- mit einem Schlauchkörper (2) aus thermoplastischem Polyurethan (TPU),
- wobei der Schlauchkörper (2) eine Shore-Härte aufweist, die größer ist als 60D
- mit einer Wandstärke (WD), die über eine Schlauchlänge von 30 cm um weniger als 0,02 mm von einer Soll-Wandstärke abweicht,
- mit einer Wandstärke (WD), die kleiner ist als 0,07 mm, und
- mit einer Durchschlagsfestigkeit, die mindestens 10 kV beträgt.

3. Wärmetauscherschlauch (1) nach Anspruch 2, **dadurch gekennzeichnet, dass** die Shore-Härte des Schlauchkörpers (2) größer ist als 70 D.

4. Wärmetauscherschlauch (1) nach Anspruch 3, **dadurch gekennzeichnet, dass** die Shore-Härte des Schlauchkörpers (2) im Bereich von 80 D liegt.

5. Wärmetauscherkörper für einen Wärmetauscher eines Oxygenators mit einer Mehrzahl von Abschnitten eines Wärmetauscherschlauchs (1) nach einem der Ansprüche 2 bis 4.

6. Wärmetauscher für einen Oxygenator
- mit einem rohrförmigen Grundkörper,
- mit einem im Grundkörper angeordneten Wärmetauscherkörper nach Anspruch 5.

## Claims

1. Method for producing a tube body (2) for a heat exchanger tube (1), comprising the following steps:
- producing a thermoplastic polyurethane plastic melt,
- filtering of the plastic melt to filter out gel particles contained in the melt,
- extruding the melt into a tube,
- cooling of the extruded tube,
- assembling the tube body (2),
wherein the tube body (2) has a Shore hardness greater than 60D, with a wall thickness (WD) that deviates from a nominal wall thickness by less than 0.02 mm over a tube length of 30 cm.

2. Heat exchanger tube (1) for a heat exchanger of an oxygenator, produced according to the method of claim 1,
- comprising a tube body (2) made of thermoplastic polyurethane (TPU),
- wherein the tube body (2) has a Shore hardness greater than 60D
- with a wall thickness (WD) which deviates from a nominal wall thickness by less than 0.02 mm over a tube length of 30 cm,
- with a wall thickness (WD) which is less than 0.07 mm, and
- with a dielectric strength which is at least 10 kV.

3. Heat exchanger tube (1) according to claim 2, **characterized in that** the Shore hardness of the tube body (2) is greater than 70 D.

4. Heat exchanger tube (1) according to claim 3, **characterized in that** the Shore hardness of the tube body (2) is in the range of 80 D.

5. Heat exchanger body for a heat exchanger of an oxygenator comprising a plurality of sections of a heat exchanger tube (1) according to any one of claims 2 to 4.

6. Heat exchanger for an oxygenator
- comprising a tubular base body,
- comprising a heat exchanger body according to claim 5 arranged in the base body.

## Revendications

1. Procédé de fabrication d'un corps de tuyau (2) pour un tuyau d'échangeur de chaleur (1), comprenant les étapes suivantes :
- préparation d'une masse de matière plastique fondue polyuréthane thermoplastique,
- filtration de la masse de matière plastique fondue afin de filtrer les particules de gel contenues dans la matière fondue,
- extrusion de la matière fondue en un tuyau,
- refroidissement du tuyau extrudé,
- assemblage du corps de tuyau (2),
dans lequel le corps de tuyau (2) a une dureté Shore supérieure à 60D, avec une épaisseur de paroi (WD) qui s'écarte de moins de 0,02 mm de l'épaisseur de paroi nominale sur une longueur de tuyau de 30 cm.

2. Tuyau d'échangeur de chaleur (1) pour un échangeur de chaleur d'un oxygénateur, fabriqué selon le procédé de la revendication 1,
- avec un corps de tuyau (2) en polyuréthane thermoplastique (TPU),
- dans lequel le corps de tuyau (2) a une dureté Shore supérieure à 60D,
- avec une épaisseur de paroi (WD) qui s'écarte de moins de 0,02 mm de l'épaisseur de paroi nominale sur une longueur de tuyau de 30 cm,
- avec une épaisseur de paroi (WD) qui est inférieure à 0,07 mm, et
- avec une rigidité diélectrique qui est d'au moins 10 kV.

3. Tuyau d'échangeur de chaleur (1) selon la revendication 2, **caractérisé en ce que** la dureté Shore du corps de tuyau (2) est supérieure à 70 D.

4. Tuyau d'échangeur de chaleur (1) selon la revendication 3, **caractérisé en ce que** la dureté Shore du corps de tuyau (2) est dans la gamme de 80 D.

5. Corps d'échangeur de chaleur pour un échangeur de chaleur d'un oxygénateur, comprenant une pluralité de sections d'un tuyau d'échangeur de chaleur (1) selon l'une quelconque des revendications 2 à 4.

6. Échangeur de chaleur pour un oxygénateur
- avec un corps de base tubulaire,
- avec un corps d'échangeur de chaleur selon la revendication 5 disposé dans le corps de base.
